(19) Europäisches Patentamt
European Patent Office
Office européen des brevets

(11) EP 0 752 999 B1

(12) **EUROPÄISCHE PATENTSCHRIFT**

(45) Veröffentlichungstag und Bekanntmachung des Hinweises auf die Patenterteilung:
**28.10.1998 Patentblatt 1998/44**

(21) Anmeldenummer: **95914285.2**

(22) Anmeldetag: **22.03.1995**

(51) Int Cl.[6]: **C07F 9/38**, A61K 31/66, C07F 9/572

(86) Internationale Anmeldenummer:
**PCT/EP95/01062**

(87) Internationale Veröffentlichungsnummer:
**WO 95/26358 (05.10.1995 Gazette 1995/42)**

(54) **NEUE 2.4-DIPHOSPHONOGLUTARSÄUREDERIVATE, VERFAHREN ZU DEREN HERSTELLUNG UND DIESE VERBINDUNGEN ENTHALTENDE ARZNEIMITTEL**

2,4-DIPHOSPHONOGLUTARIC ACID DERIVATIVES, METHODS OF PREPARING SUCH COMPOUNDS AND DRUGS CONTAINING THEM

NOUVEAUX DERIVES DE L'ACIDE 2,4-DIPHOSPHONOGLUTARIQUE, LEUR PROCEDE DE FABRICATION ET MEDICAMENTS LES RENFERMANT

(84) Benannte Vertragsstaaten:
**AT BE CH DE DK ES FR GB GR IE IT LI LU NL PT SE**

(30) Priorität: **26.03.1994 DE 4410601**

(43) Veröffentlichungstag der Anmeldung:
**15.01.1997 Patentblatt 1997/03**

(73) Patentinhaber: **BOEHRINGER MANNHEIM GMBH**
**68298 Mannheim (DE)**

(72) Erfinder:
- **ZIMMERMANN, Gerd**
  **D-68309 Mannheim (DE)**
- **ESSWEIN, Angelika**
  **D-78224 Singen (DE)**
- **TSAKLAKIDIS, Christos**
  **D-69469 Weinheim (DE)**
- **BAUSS, Frieder**
  **D-67141 Neuhofen (DE)**

(74) Vertreter: **Weber, Manfred, Dr.**
**c/o Boehringer Mannheim GmbH,**
**Patentabteilung,**
**Sandhoferstrasse 116**
**68298 Mannheim (DE)**

(56) Entgegenhaltungen:
**WO-A-93/12122** **WO-A-93/24131**

- **JOURNAL OF ORGANIC CHEMISTRY, Bd.25, 1960, EASTON US Seiten 1232 - 1234 SAUL PATAI 'Condensation of triethyl phosphonoacetate with aromatic aldehydes' in der Anmeldung erwähnt**
- **CHEMICAL ABSTRACTS, vol. 094, no. 4, 26. Januar 1981, Columbus, Ohio, US; abstract no. 022889, 'Silver halide color photographic developers' & JP,A,55 067 747 (KONISHIROKU PHOTO INDUSTRY CO., LTD.;JAPAN) 22 Mai 1980**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 752 999 B1

Printed by Jouve, 75001 PARIS (FR)

## Beschreibung

Die vorliegende Erfindung betrifft neue 2.4-Diphosphonoglutarsäurederivate, Verfahren zu deren Herstellung sowie Arzneimittel, die diese Substanzen enthalten.

In Angew. Chemie 92, 43 (1983) und Can. J. Chem. 60, 840 (1982) ist der 2.4-Diphosphonoglutarsäurehexaethylester beschrieben, in J. Org. Chem. 25, 1232 (1960) ist der 2.4-Diphosphono-3-phenylglutarsäurehexaethylester angeführt, eine pharmakologische Wirkung dieser Verbindungen ist jedoch nicht bekannt.

Es wurde nun gefunden, daß in 2-Stellung substituierte 2.4-Diphosphonoglutarsäuren eine ausgezeichnete Wirkung auf den Calcium-Stoffwechsel zeigen und damit zur breiten Behandlung von Calciumstoffwechselstörungen geeignet sind.

Sie lassen sich vor allem sehr gut dort einsetzen, wo der Knochenauf- und -abbau gestört ist, d. h. sie sind geeignet zur Behandlung von Erkrankungen des Skelettsystems, wie z. B. Osteoporose, Morbus Paget, Morbus Bechterew, Paradontosen u.a.

Aufgrund dieser Eigenschaften finden sie aber auch Verwendung in der Therapie der Urolithiasis und zur Verhinderung heterotoper Ossifikationen. Durch ihre Beeinflussung des Calciumstoffwechsels bilden sie weiterhin eine Grundlage für die Behandlung der rheumatoiden Arthritis, der Osteoarthritis und der degenerativen Arthrose. Durch die ausgesprochen gute Affinität zum Knochenmineral eignen sich die 2,4-Diphosphonoglutarsäure und ihre Derivate auch hervorragend, um pharmakologisch wirksame Verbindungen direkt an den Knochen zu transportieren.

Gegenstand der vorliegenden Erfindung sind Verbindungen der allgemeinen Formel I

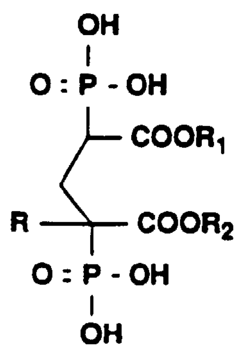

(I),

in der

R niederes Alkyl, das gegebenenfalls durch Hydroxy, Alkoxy, Amino, Dialkylamino, Alkylmercapto, Alkylsulfinyl, Alkansulfonyl, Cycloalkyl, Aryl oder einen heterocyclischen Ring substituiert sein kann, niederes Alkenyl, Cycloalkyl, Cycloalkenyl, Aryl oder einen heterocyclischen Ring bedeutet und

$R_1$ und $R_2$ unabhängig voneinander jeweils Wasserstoff, niederes Alkyl, Cycloalkyl, Aryl oder Arylmethyl sein können,

sowie deren pharmakologisch unbedenkliche Salze.

Niederes Alkyl soll in allen Fällen eine geradkettige oder verzweigte $C_1$-$C_6$-Alkylgruppe, wie z. B. Methyl, Ethyl, Propyl, Isopropyl, Butyl, Isobutyl, Pentyl oder Hexyl, insbesondere Methyl, Ethyl, Isopropyl, Butyl und Hexyl darstellen. Niederes Alkenyl bedeutet ungesättigte Reste mit 3 - 6 Kohlenstoffatomen, wie z. B. Allyl, But-2-enyl, Hexa-2.4-dienyl, vor allem jedoch Allyl.

Cycloalkyl bedeutet einen 3- bis 7-gliedrigen Ring, wie den Cyclopropyl-, Cyclobutyl-, Cyclopentyl-, Cyclohexyl- oder den Cycloheptylring, vorzugsweise den Cyclopentyl- und Cyclohexylring.

Cycloalkenyl soll einen ungesättigten 5-7-Ring darstellen, wie den Cyclopentenyl-, Cyclohexenyl- und Cycloheptenylring.

Unter Alkoxy versteht man Reste wie den Methoxy-, Ethoxy-, Propoxy-, Isopropoxy-, Butoxy- oder Pentoxyrest, insbesondere den Methoxy-, Ethoxy-, Isopropoxy- und Butoxyrest.

Dialkylamino soll eine Aminogruppe mit zwei gleichen oder verschiedenen $C_1$-$C_5$-Alkylresten, wie z. B. Dimethylamino, Methylpropylamino. Methylpentylamino, Diethylamino oder Dipropylamino, vor allem Dimethylamino, Methylpropylamino und Methylpentylamino darstellen.

Unter Alkylmercapto, Alkylsulfinyl bzw. Alkansulfonyl sind vorzugsweise Methylmercapto, Methylsulfinyl bzw. Methansulfonyl zu verstehen.

Aryl bedeutet den Phenyl- oder Naphthylrest, der gegebenenfalls ein- oder mehrfach durch niederes Alkyl, Halogen, Hydroxy, Alkoxy, Amino, Dialkylamino, Alkylmercapto, Alkylsulfinyl, Alkansulfonyl, Carboxamido, das ein- oder zweifach durch niederes Alkyl substituiert sein kann, Carboxyl, Alkoxycarbonyl oder Cyano substituiert sein kann.

Unter einem heterocyclischen Ring ist ein 5- bis 7gliedriger, gesättigter oder ungesättigter Ring, wie z. B. der Pyrrolidin-, Piperidin-, Azepin-, Tetrahydrofuran-, Tetrahydropyran-, Morpholin-, Dioxan-, Furan-, Thiophen-, Pyridin-, Pyrazol-, Imidazol-, Thiazol-, Oxazol-, Pyrimidin- oder Pyrazinring, vorzugsweise der Pyrrolidin-, Piperidin-, Morpholin-, Furan-, Thiophen-, Pyridin- und Imidazolring zu verstehen, wobei das Ringsystem ein- oder mehrfach durch Halogen, niederes Alkyl oder Alkoxy substituiert sein kann.

Halogen soll Fluor, Chlor, Brom und Jod, insbesondere Fluor und Chlor darstellen.

Verbindungen der allgemeinen Formel **I** enthalten mindestens zwei asymmetrische Kohlenstoffatome; daher sind auch optisch aktive Verbindungen der allgemeinen Formel **I** Gegenstand der vorliegenden Anmeldung.

Verbindungen der allgemeinen Formel **I** werden nach an sich bekannten Verfahren hergestellt, vorzugsweise dadurch, daß man eine Verbindung der allgemeinen Formel **II**,

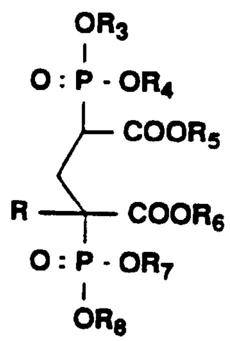

(II),

in der R die oben angegebene Bedeutung hat und $R_3$, $R_4$, $R_5$, $R_6$, $R_7$ und $R_8$ jeweils unabhängig voneinander für Niederalkyl, Cycloalkyl, Aryl oder Arylmethyl stehen, in einer Weise verseift, daß entweder alle Esterbindungen hydrolysiert werden oder durch geeignete Reagentien eine Teilverseifung erreicht wird.

Eine vollständige Verseifung von Verbindungen der allgemeinen Struktur **II** erreicht man durch Kochen mit wässrigen Mineralsäuren, vorzugsweise mit 6 N Salzsäure.

Die selektive Verseifung der Phosphonestergruppen läßt sich mit Halogentrialkylsilanen, vorzugsweise Brom- oder Jodtrimethylsilan in inerten Lösungsmitteln z.B. Dichlormethan bei Temperaturen zwischen - 50 °C und + 50 °C, vorzugsweise bei 0 - 20 °C erreichen. Liegen in den Verbindungen der Formel **II** Benzylester vor, so lassen sich diese selektiv durch katalytische Hydrierung z. B. über Palladium/Kohle spalten.

Die Verbindungen der allgemeinen Formel **II** sind neu und Gegenstand der Erfindung. Sie lassen sich herstellen

a) analog dem in Can. J. Chem. 60, 840 (1982) beschriebenen Verfahren indem man eine Verbindung der Struktur **III** in der die Reste R, $R_6$, $R_7$, und $R_8$ die oben angegeben Bedeutung haben, in Gegenwart eine starken Base, vorzugs- weise Natriumhydrid in Tetrahydrofuran oder Natriummethylat in Methanol, mit einem Phosphonoacrylester der allgemeinen Formel **IV**, in der $R_3$, $R_4$, und $R_5$ die oben angegebene Bedeutung haben, umsetzt,

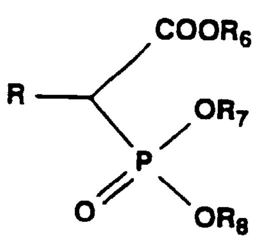

**III**

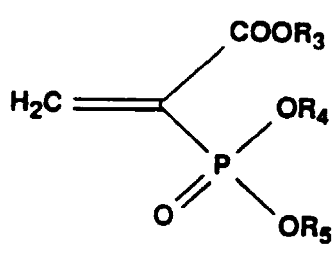

**IV**

oder

b) eine Verbindung der allgemeinen Formel **V** in der R, $R_5$ und $R_6$ die oben angegebene Bedeutung haben und X für eine Abgangsgruppe wie z. B. Halogen , vorzugsweise Brom oder Jod oder für eine Gruppe -O-$SO_2$-Z , in der Z für Methyl oder Aryl z. B. Phenyl oder p-Nitrophenyl steht analog dem in Angew. Chem. 92, 43 (1980) beschriebenen Verfahren mit einem Trialkylphosphit umsetzt.

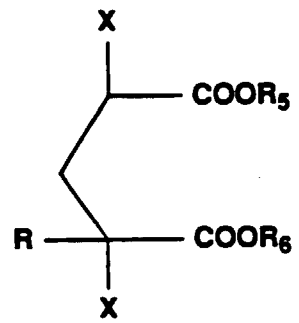

(V),

Die Verbindungen der allgemeinen Formeln **III**, **IV** und **V** sind beschrieben oder lassen sich nach analogen Verfahren herstellen.

Eine Auftrennung der Diastereomerengemische von Verbindungen der Formel **I** odere deren Vorstufen, sofern mindestens zwei asymmetrische C-Atome vorliegen, kann durch bekannte Verfahren wie Kristallisation oder durch chromatographische Verfahren erfolgen. Die Bestimmung der absoluten Konfiguration von Enantiomeren ist durch Röntgenstrukturanalyse möglich.

Als pharmakologisch verträgliche Salze werden vor allem Mono- bzw. Dialkali- oder Ammoniumsalze verwendet, die in üblicher Weise, z. B. durch Titration der Verbindungen mit anorganischen Basen. wie z. B. Natrium- oder Kaliumhydrogencarbonat, Natronlauge, Kalilauge, wäßrigem Ammoniak oder Aminen, wie z. B. Trimethyl- oder Triethylamin, hergestellt werden.

Die Salze werden in der Regel durch Umfällen aus Wasser/Aceton gereinigt. Es lassen sich jedoch auch Calcium-, Magnesium- oder Zinksalze verwenden, die in der Regel in Wasser schwer löslich, bisweilen jedoch auch leicht löslich sind. Diese Salze können dadurch hergestellt werden, daß man ein Calcium-, Zink- oder Magnesiumsalz, wie z. B. Calciumcarbonat, Calciumhydrogencarbonat, Calciumhydroxid, Calciumchlorid, Zinkcarbonat, Zinkhydrogencarbonat, Zinkhydroxid, Zinkchlorid, Magnesiumcarbonat, Magnesiumhydrogencarbonat, Magnesiumhydroxid oder Magnesiumchlorid als wäßrige Lösung oder Suspension im Verhältnis 1 : 1 oder 2 : 1 mit einer wäßrigen Lösung oder Suspension der 2.4-Diphosphonoglutarsäure bei 20 - 120 °C rühren läßt, den Niederschlag anschließend absaugt oder die wäßrige Lösung anschließend einengt.

Die erfindungsgemäßen neuen Substanzen der Formel **I** und ihre Salze können in jeder für das Erreichen einer effektiven Dosis geeigneten flüssigen oder festen Form z. B. oral, rektal, topisch, parenteral, subkutan, okular, nasal, bukkal, intravenös und transdermal appliziert werden. Hierbei kommen alle üblichen Applikationsformen in Frage, beispielsweise Tabletten, Kapseln, Dragees, Sirupe, Lösungen, Suspensionen etc. Als Injektionsmedium kommt vorzugsweise Wasser zur Anwendung, welches die bei Injektionslösungen üblichen Zusätze wie Stabilisierungsmittel, Lösungsvermittler und Puffer enthält.

Derartige Zusätze sind z. B. Tartrat- und Citrat-Puffer, Ethanol, Komplexbildner (wie Ethylendiamintetraessigsäure und deren nichttoxische Salze), hochmolekulare Polymere (wie flüssiges Polyethylenoxid) zur Viskositätsregelung. Flüssige Trägerstoffe für Injektionslösungen müssen steril sein und werden vorzugsweise in Ampullen abgefüllt. Feste Trägerstoffe sind z. B. Stärke, Lactose, Mannit, Methylcellulose, Talkum, hochdisperse Kieselsäuren, höhermolekulare Fettsäuren (wie Sterinsäure), Gelatine, Agar-Agar, Calciumphosphat, Magnesiumstearat, tierische und pflanzliche Fette, feste hochmolekulare Polymere (wie Polyethylenglykole); für orale Applikation geeignete Zubereitungen können gewünschtenfalls Geschmacks- und Süßstoffe enthalten.

Die Dosierung kann von verschiedenen Faktoren, wie Applikationsweise, Spezies, Alter und/oder individuellem Zustand abhängen. Die täglich zu verabreichenden Dosen liegen bei etwa 10 - 1000 mg/Mensch, vorzugsweise bei 100 - 500 mg/Mensch und können auf einmal oder mehre Male verteilt eingenommen werden.

Eine pharmazeutische Formulierung dieser 2,4-Diphosphonoglutarsäuren kann auch intermittierend verabreicht werden. Einer Gabe von z. B. 1 - 90 Tagen kann eine substanzfreie Zeit von z. B. 30 - 180 Tagen folgen, wonach sich wieder eine Substanzgabe von 1 - 90 Tagen anschließen kann.

Bevorzugt im Sinne der vorliegenden Erfindung sind außer den in den Beispielen genannten Verbindungen und durch Kombination aller in den Ansprüchen genannten Bedeutungen der Substituenten ableitbaren Verbindungen die folgenden 2.4-Diphosphonoglutarsäurederivate sowie deren Natrium-, Kalium-, Ammonium-, Calcium-, Zink- und Magnesiumsalze, Carbonsäuremethyl-,-ethyl- oder -benzylester.

2.4-Diphosphono-2-ethyl-glutarsäure
2.4-Diphosphono-2-isopropyl-glutarsäure
2.4-Diphosphono-2-hexyl-glutarsäure
2.4-Diphosphono-2-hydroxymethyl-glutarsäure
2.4-Diphosphono-2-(3-hydroxypropyl)-glutarsäure
2.4-Diphosphono-2-(4-hydroxybutyl)-glutarsäure
2.4-Diphosphono-2-(2-methoxyethyl)-glutarsäure
2.4-Diphosphono-2-butoxymethyl-glutarsäure
2.4-Diphosphono-2-ethoxymethyl-glutarsäure
2.4-Diphosphono-2-(2-aminoethyl)-glutarsäure
2.4-Diphosphono-2-(4-aminobutyl)-glutarsäure
2.4-Diphosphono-2-(2-**N**-methyl-**N**-pentyl-aminoethyl)-glutarsäure
2.4-Diphosphono-2-(2-dimethylaminoethyl)-glutarsäure
2.4-Diphosphono-2-(2-**N**-methyl-**N**-propylaminoethyl)-glutarsäure
2.4-Diphosphono-2-methylmercaptomethyl-glutarsäure
2.4-Diphosphono-2-methylsulfinylmethyl-glutarsäure
2.4-Diphosphono-2-methansulfonylmethyl-glutarsäure
2.4-Diphosphono-2-(2-cyclohexylethyl)-glutarsäure
2.4-Diphosphono-2-benzyl-glutarsäure
2.4-Diphosphono-2-(2-phenylethyl)-glutarsäure
2.4-Diphosphono-2-(4-chlorbenzyl)-glutarsäure
2.4-Diphosphono-2-[2-(4-methylphenyl)ethyl]-glutarsäure
2.4-Diphosphono-2-(3.4-dichlorbenzyl)-glutarsäure
2.4-Diphosphono-2-[2-(5 Chlor-2-methoxyphenyl)ethyl]-glutarsäure
2.4-Diphosphono-2-[2-(2-pyridyl)ethyl]-glutarsäure
2.4-Diphosphono-2-[2-(3-pyridyl)ethyl]-glutarsäure
2.4-Diphosphono-2-[2-(4-pyridyl )ethyl]-glutarsäure
2.4-Diphosphono-2-(2-thenyl)-glutarsäure
2.4-Diphosphono-2-(3-thenyl)-glutarsäure
2.4-Diphosphono-2-(2-furfuryl)-glutarsäure
2.4-Diphosphono-2-[2-(2-imidazolyl)ethyl]-glutarsäure
2.4-Diphosphono-2-(1-imidazolylmethyl)-glutarsäure
2.4-Diphosphono-2-(1-pyrrolidinylmethyl)-glutarsäure
2.4-Diphosphono-2-(1-piperidinylmethyl)-glutarsäure
2.4-Diphosphono-2-(1-morpholinylmethyl)-glutarsäure
2.4-Diphosphono-2-allyl-glutarsäure
2.4-Diphosphono-2-(but-2-enyl)-glutarsäure
2.4-Diphosphono-2-cyclopentyl-glutarsäure
2.4-Diphosphono-2-cycloheptyl-glutarsäure
2.4-Diphosphono-2-(3-pyridyl)-glutarsäure
2.4-Diphosphono-2-(6-methyl-2-pyridyl)-glutarsäure
2.4-Diphosphono-2-(5-chlor-2-pyridyl)-glutarsäure
2.4-Diphosphono-2-(2 methyl-4-pyridyl)-glutarsäure
2.4-Diphosphono-2-(2-furyl)-glutarsäure
2.4-Diphosphono-2-(3-thienyl)-glutarsäure
2.4-Diphosphono-2-(2-imidazolyl)-glutarsäure
2.4-Diphosphono-2-(4-imidazolyl)-glutarsäure
2.4-Diphosphono-2-(2-methyl-4-imidazolyl)-glutarsäure
2.4-Diphosphono-2-(2-methoxyphenyl)-glutarsäure
2.4-Diphosphono-2-(5-chlor-2-methoxyphenyl)-glutarsäure
2.4-Diphosphono-2-(3.4-dichlorphenyl)-glutarsäure
2.4-Diphosphono-2-(4-isopropoxyphenyl)-glutarsäure
2.4-Diphosphono-2-(4-methylphenyl)-glutarsäure
2.4-Diphosphono-2-(2.4-dimethylphenyl)-glutarsäure
2.4-Diphosphono-2-(3-hydroxyphenyl)-glutarsäure
2.4-Diphosphono-2-(4-dimethylaminophenyl)-glutarsäure

2.4-Diphosphono-2-(4-**N**-methyl-**N**-pentylaminophenyl)-glutatsäure
2.4-Diphosphono-2-(4-methylmercaptophenyl)-glutarsäure
2.4-Diphosphono-2-(4-methylsulfinylphenyl)-glutarsäure
2.4-Diphosphono-2-(4-methylsulfonylphenyl)-glutarsäure
2.4-Diphosphono-2-(4-carboxyphenyl)-glutarsäure
2.4-Diphosphono-2-(4-ethoxycarbonylphenyl)-glutarsäure
2.4-Diphosphono-2-(4-carboxamidophenyl)-glutarsäure
2.4-Diphosphono-2-(4-**N**,**N**-dimethylcarboxamidophenyl)-glutarsäure
2.4-Diphosphono-2-(3-cyanophenyl)-glutarsäure
2.4-Diphosphono-2-(4-cyanophenyl)-glutarsäure
2.4-Diphosphono-2-(1-naphthyl)-glutarsäure
2.4-Diphosphono-2-(2-naphthyl)-glutarsäure
2.4-Diphosphono-2-(3-indolyl)-glutarsäure
2.4-Diphosphono-2-(3-indolylmethyl)-glutarsäure
2.4-Diphosphono-2-(2.3-dihydroxypropyl)-glutarsäure

Die nachfolgenden Beispiele zeigen einige der Verfahrensvarianten, die zur Synthese der erfindungsgemäßen Verbindungen verwendet werden können. Sie sollen jedoch keine Einschränkung des Erfindungsgegenstandes darstellen. Die Struktur der Verbindungen wurde durch $^{1}H$-, $^{31}P$ und gegebenenfalls durch $^{13}C$- Kernresonanzspektroskopie gesichert. Die Reinheit der Substanzen wurde mittels Elementaranalyse (C,H,N,P, gegebenenfalls Na) sowie dünnschichtchromatographisch oder durch Dünnschichtelektrophorese (Cellulose, Oxalatpuffer pH 4) bestimmt.

**Beispiel 1**

2,4-Diphosphono-2-phenyl-glutarsäure

Zu einer Suspension von 0.75 g Natriumhydrid in 30 ml trockenem Tetrahydrofuran tropft man eine Lösung von 9 g α-Phosphonophenylessigsäuretriethylester. Man rührt 30 min bei Raumtemperatur und tropft anschließend 4.9 g 2-Phosphonoacrylsäuretrimethylester hinzu. Das Reaktionsgemisch wird eine Stunde bei 30 - 40 °C gerührt und schließlich noch 2 Stunden bei 60 °C. Das Gemisch wird eingedampft, der Rückstand in Essigester aufgenommen und die organische Phase mit wässriger Ammoniumchloridlösung ausgeschüttelt. Die Essigesterphase wird getrocknet und eingedampft. Das Rohprodukt (10.5 g) wird säulenchromatographisch an Kieselgel mit Essigester/Isohexan 1:2, Essigester/Methanol 95:5 und 80:20 gereinigt. Das gewünschte Produkt befindet sich in den Essigester/Methanol 8:2 Fraktionen. Man erhält 4.5 g öligen 2,4-Diphosphono-2-phenylglutarsäure-diethyl-trimethylester (Diastereomerengemisch). Das so erhaltene Zwischenprodukt wird mit 50 ml 6 N HCl 36 Stunden unter Rückfluß gekocht und die Reaktionsmischung eingedampft. Der Rückstand wird durch Chromatographie an dem Adsorberharz HP 20SS (Fa. Mitsubishi) mit Wasser als Elutionsmittel gereinigt. Man erhält zwei Produkte, die sich nach $^{1}H$-NMR und Massenspektrum als die R,R /S,S bzw. R,S/S,R- Diastereomere der 2.4-Diphosphono-2-phenyl-glutarsäure erweisen.

Man erhält:

1.5 g Diastereomeres 1 der 2,4-Diphosphono-2-phenyl-glutarsäure
$^{1}H$-NMR($D_2O$) δ (ppm): 7.5 (m,5H); 3.05 (m,3H)

0.6 g Diastereomeres 2 der 2,4-Diphosphono-2-phenyl-glutarsäure
$^{1}H$-NMR ($D_2O$) δ (ppm): 7.5 (m,2H); 6.9 (m,3H); 3.27 (m, 1H); 2.85 (m,2H)

**Beispiel 2**

Analog Beispiel 1 erhält man ausgehend von 2-Phosphonopropionsäuretriethylester 2,4-Diphosphono-2-methylglutarsäure als Gemisch der Diastereomeren.
$^{1}H$-NMR ($D_2O$): δ (ppm) 2.1-3.1 (m, 3H), 1.4, 1.35 (2d, 3H)

**Beispiel 3**

Analog Beispiel 1 erhält man ausgehend von 2-Phosphonohexansäuretriethylester 2,4-Diphosphono-2-butylglutarsäure in Form von 2 Produkten, die sich nach $^{1}H$-NMR und Massenspektrum als die R,R/S,S bzw. R,S/S,R-Diastereomere erweisen.

Diastereomeres 1
$^{1}H$-NMR ($D_2O$): δ (ppm) 3.22 (dd, 1H); 2.68 (m, 1H) 2.35 (m, 1H); 1.85 (m, 2H); 1.35 (m, 4H); 0.9 (t, 3H)

Diastereomeres 2
$^1H$-NMR ($D_2O$): δ (ppm) 3.35 (dd, 1H); 2.65 (m, 1H); 2.28 (m, 2H); 1.37 (m, 4H); 0.88 (t, 3H)

**Beispiel 4**

2.4-Diphosphono-2-pyrrolidinopropylglutarsäure

4.1. 2.2 g Phosphonoessigsäuretriethylester werden in 20 ml Dimethylformamid gelöst und mit 240 mg Natriumhydrid versetzt. Man rührt das Gemisch 30 min lang und gibt dann 2.5 g 1-Brom-3-tert.-butyldimethylsilyloxypropan hinzu. Nach 4stündi- gem Rühren wird das Reaktionsgemisch eingedampft, der Rückstand mit Ammo- niumchloridlösung versetzt und das Produkt mit Ether extrahiert. Zur Reinigung wird das Rohprodukt über Kieselgel chromatographiert (Laufmittel Essigester / Isohexan 2:1). Man erhält 1.7 g 5-tert. Butyldimethylsilyloxy-2-phosphonovaleriansäuretriethylester.

4.2. Analog Beispiel 1 setzt man das unter 4.1. erhaltene Produkt mit dem 2-Phosphonoacrylsäuretrimethylester um. Auf die saure Verseifung der Esterfunktionen wird verzichtet, statt dessen wird die Silylschutzgruppe durch Einwirkung von 1M HF in Acetonitril abgespalten. Man erhält so 2,4-Diphosphono-2-hydroxypropylglutarsäure-triethyl-trimethylester.

4.3. Die unter 4.2. erhaltene Hydroxyverbindung (1.5 g) wird in 20 ml Methylenchlorid gelöst und unter Eiskühlung in Gegenwart von 0.55 ml Triethylamin mit 0.27 ml Methansulfonylchlorid umgesetzt. Man rührt 30 min und schüttelt die Methylenchloridphase mit Natriumbicarbonatlösung aus. Nach dem Eindampfen der organischen Phase verbleiben 1.6 g der entsprechenden O-Methansulfonylverbinbindung, die mit 5 ml Pyrrolidin versetzt wird. Man läßt über Nacht stehen, setzt Äther zu und filtriert. Das Filtrat wird eingedampft. Es verbleiben 1.5 g der entsprechenden Pyrolidino-Verbindung, die in 20 ml 6N Salzsäure aufgenommen und 24 Std. unter Rückfluß gekocht werden. Die Reaktionsmischung wird mit Essigester extrahiert, die wässrige Phase gekohlt und eingedampft. Der Rückstand wird in Wasser gelöst, die Lösung mit 1N NaOH neutralisiert (pH 7) und eingedampft. Das verbleibende Produkt wird mit Aceton verrührt, der Niederschlag abgesaugt und getrocknet. Man erhält 1.15 g der Titelverbindung als Tetranatriumsalz (Diastereomerengemisch).
$^1H$-NMR ($D_2O$): δ (ppm) 4.3: 4.05 (m, 1H); 3.8 (m, 3H) 3.3 (m, 6H); 2.2 (m, 8H)

**Beispiel 5**

2-Aminobutyl-2,4-diphosphonoglutarsäure

3.7 g 2-Brom-6- phthalimidohexansäureethylester werden zu 1.9 ml, auf 145°C erhitztem Triethylphosphit gegeben und die Mischung 4 Std. bei einer Badtemperatur von 160°C gerührt. Das überschüssige Triethylphosphit wird abdestilliert und der Rückstand über Kieselgel mit Essigester chromatographiert. Man erhält 0.9 g 2-Phosphono-6-phthalimidohexansäuretriethlyester, der analog Beispiel 1 mit Phosphonoacrylsäuretrimethylester umgesetzt wird. Das Produkt wird ebenfalls analog Beispiel 1 durch Kochen mit Salzsäure verseift, die entstandene Phthalsäure mit Essigester extrahiert und das Produkt aus der wässrigen Phase isoliert. Durch Neutralisation der wässrigen Lösung auf pH 7, eindampfen und verreiben des Rückstandes mit Aceton erhält man das Tetranatriumsalz der 2-Aminobutyl-2,4-diphosphono-glutarsäure als Diastereomerengemisch.
$^1H$-NMR ($D_2O$): δ (ppm) 4.2, 3.7 (m, 1H); 3.1 (m, 2H); 2.5 (m, 2H); 1.7 (m, 6H)

**Beispiel 6**

**Pharmakologischer Versuchsbericht**

Inhibierung der nichtstimulierten Knochenresorption durch die Bestimmung der [$^3H$]-Tetracyclin-Ausscheidung im Urin:

Von Geburt an erhalten Ratten zweimal wöchentlich eine Injektion mit steigenden Mengen einer Lösung aus 3.7 x $10^5$ Bq/ml (10 μCi/ml) [7-$^3H$]-Tetracyclin ([$^3H$]TC); New England Nuclear, Boston, MA) der spezifischen Aktivität 679 mCi/mmol. Das Volumen pro Injektion wird von 50 μl/Woche auf 250 μl in der fünften Woche gesteigert und für eine weitere Woche beibehalten. Die Gesamtmenge an appliziertem [$^3H$]TC betrug 20 μCi pro Ratte. 51 Tage alte Ratten werden in individuelle Stoffwechselkäfige umgesetzt und erhalten eine Gruppenfütterung mit Futter, das 0.5 % Ca und 0.35 % P enthält. Dieses Futter wurde durch Zusatz von entsprechenden Mengen Calcium- und Phosphatgehalt (SODI 2134, Klingenthalmühle) hergestellt. Während der gesamten Versuchsdauer erhielten die Tiere Aqua dest. ad libitum.

Nach 61 Tagen wurde mit dem Sammeln des 24 h-Urins begonnen. Der Urin wurde täglich um 11 Uhr gesammelt; zu dieser Zeit erhielten die Tiere auch Futter. Am sechsten Tag nach Beginn der Urinsammlung erhielten die Tiere zwei subkutane Injektionen mit Substanz (um 8 Uhr bzw. 17 Uhr). Die Inhibierung der Knochenresorption wird durch die verminderte [3H]-Tetracyclinausscheidung am Tag 8 nach Beginn der Urinsammlung angegeben. Das $^3$H-TC im Urin wurde durch Flüssigkeitsszintillation bestimmt. indem zu 1 ml Urin 10 ml des Szintillators Pico-Fluor 30 (packard International, Zürich, Schweiz) zugesetzt wurde.

$$\% \text{ rel. Inhibierung} = \frac{[Xcpm_{Tag5} - (Ccpm_{Tag8} - Ccpm_{Tag5})] - Xcpm_{Tag8}}{[Xcpm_{Tag5} - (Ccpm_{Tag8} - Ccpm_{Tag5})]} \times 100\ \%$$

X = Behandelte Tiere
C = Kontrolltiere

Tabelle

| Beispiel-Nr. | % rel. Inhibierung/Dosierung |
|---|---|
| 4 | 37 % bei 2 x 200 mg/kg |
| 5 | 45 % bei 2 x 200 mg/kg |

**Patentansprüche**

**1.** Verbindungen der Formel I

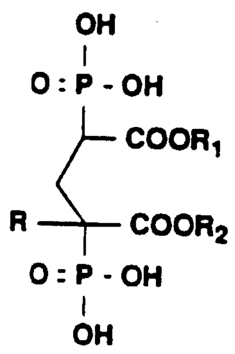

(I),

in der

R C$_1$-C$_6$-Alkyl, das gegebenenfalls durch Hydroxy, C$_1$-C$_6$-Alkoxy, Amino, Di-C$_1$-C$_6$-alkylamino, C$_1$-C$_6$-Alkylmercapto, C$_1$-C$_6$-Alkylsulfinyl, C$_1$-C$_6$-Alkansulfonyl, C$_3$-C$_7$-Cycloalkyl, Aryl oder einen heterocyclischen Ring substituiert sein kann, C$_3$-C$_6$-Alkenyl, C$_3$-C$_7$-Cycloalkyl, C$_5$-C$_7$.Cycloalkenyl, Aryl oder einen heterocyclischen Ring bedeutet und

$R_1$ und $R_2$ unabhängig voneinander jeweils Wasserstoff, C$_1$-C$_6$-Alkyl, C$_3$-C$_7$-Cycloalkyl, Aryl oder Arylmethyl sein können,

deren optisch aktisve Verbindungen sowie deren pharmakologisch verträgliche Salze.

**2.** Verbindungen der Formel I gemäß Anspruch 1, wobei Aryl einen Phenyl- oder Naphthylrest bedeutet, der gegebenenfalls ein- oder mehrfach durch niederes Alkyl, Halogen, Hydroxy, Alkoxy, Amino, Dialkylamino, Alkylmercapto, Alkylsulfinyl, Alkansulfonyl, Carboxamido, das ein- oder zweifach durch niederes Alkyl substituiert sein kann, Carboxyl, Alkoxycarbonyl oder Cyano substituiert sein kann.

**3.** Verbindungen der Formel I gemäß Anspruch 1, wobei der heterocyclische Ring ein 5 - 7gliedriger, gesättigter oder

ungesättigter Ring darstellt, der ein - oder mehrfach durch Halogen $C_1$-$C_6$-Alkyl oder $C_1$-$C_6$-Alkoxy substituiert sein kann, ausgewählt aus der Gruppe Pyrrolidin, Piperidin, Azepin, Tetrahydrofuran, Tetrahydropyran, Morpholin, Dioxan, Furan, Thiophen, Pyridin, Pyrazol, Imidazol, Thiazol, Oxazol, Pyrimidin oder Pyrazin.

**4.** Verbindung gemäß Anspruch 3, wobei der heterocyclische Ring Pyrrolidin, Piperidin, Morpholin, Furan, Thiophen, Pyridin oder Imidazol ist.

**5.** Verfahren zur Herstellung von Verbindungen der Formel I gemäß Anspruch 1 bis 4, dadurch gekennzeichnet, daß man in an sich bekannter Weise eine Verbindung der Formel II

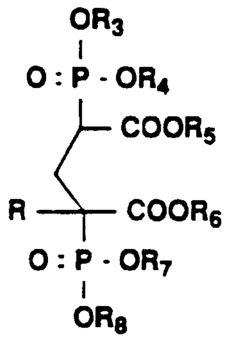

(II),

in der R die oben angegebene Bedeutung hat und $R_3$, $R_4$, $R_5$, $R_6$, $R_7$ und $R_8$ jeweils unabhängig voneinander für $C_1$-$C_6$-Alkyl, Cycloalkyl, Aryl oder Arylmethyl stehen, in einer Weise verseift, daß entweder alle Esterbindungen hydrolysiert werden oder durch geeignete Reagentien eine Teilverseifung erreicht wird,
und anschließend gewünschtenfalls von erhaltenen Diastereomeren die optisch aktiven Komponenten isoliert und erhaltenen Verbindungen gegebenenfalls in pharmakologisch unbedenkliche Salze überführt.

**6.** Verbindungen der Formel II

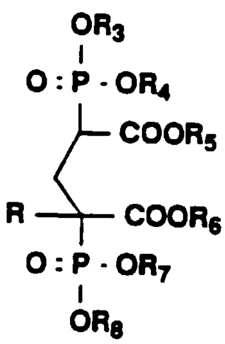

(II),

in der R die oben angegebene Bedeutung hat und $R_3$, $R_4$, $R_5$, $R_6$, $R_7$ und $R_8$ jeweils unabhängig voneinander für $C_1$-$C_6$-Alkyl, Cycloalkyl, Aryl oder Arylmethyl stehen sowie deren optisch aktive Verbindungen.

**7.** Verfahren zur Herstellung von Verbindungen der Formel II gemäß Anspruch 6, dadurch gekennzeichnet, daß man in an sich bekannter Weise entweder

a) eine Verbindung der Formel III

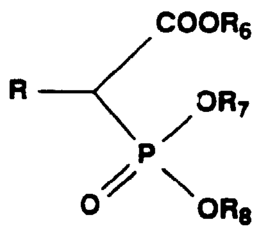

(III),

in der R, $R_6$, $R_7$ und $R_8$ die angegebene Bedeutung haben, mit einer Verbindung der Formel IV

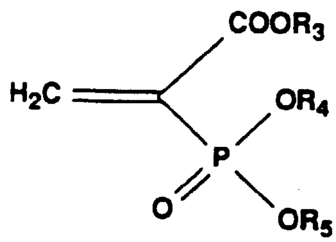

IV

(IV),

in der $R_3$, $R_4$ und $R_5$ die angegebene Bedeutung haben, umsetzt, oder

b) eine Verbindung der Formel V

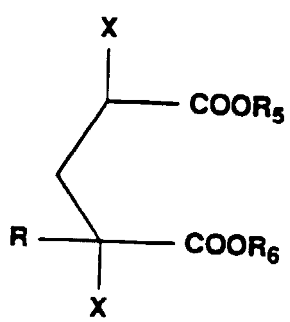

(V),

in der R, $R_5$ und $R_6$ die angegebene Bedeutung haben und X für eine Abgangsgruppe steht, mit einem Trialkylphosphit umsetzt,

und anschließend erhaltene Diastereomere in die optisch aktiven Komponenten überführt.

**8.** Arzneimittel, enthaltend eine Verbindung der Formel I gemäß Anspruch 1 bis 4 neben üblichen Träger- und Hilfsstoffen.

**9.** Verwendung von Verbindungen der Formel 1 gemäß Anspruch 1, 2, 3 oder 4 zur Herstellung von Arzneimitteln zur Behandlung von Calciumstoffwechselerkrankungen.

## Claims

**1.** Compounds of formula I

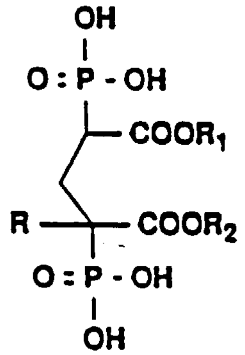

(I)

in which

R denotes, $C_1$-$C_6$ alkyl which can optionally be substituted by hydroxy, $C_1$-$C_6$ alkoxy, amino, di-$C_1$-$C_6$ alkylamino, $C_1$-$C_6$ alkylmercapto, $C_1$-$C_6$ alkylsulfinyl, $C_1$-$C_6$ alkansulfonyl, $C_3$-$C_7$ cycloalkyl, aryl or a heterocyclic ring, or it denotes $C_3$-$C_6$ alkenyl, $C_3$-$C_7$ cycloalkyl, $C_5$-$C_7$ cycloalkenyl, aryl or a heterocyclic ring and

$R_1$ and $R_2$ can each be, independently of one another, hydrogen, $C_1$-$C_6$ alkyl, $C_3$-$C_7$ cycloalkyl, aryl or arylmethyl,

their optically active compounds as well as pharmacologically acceptable salts thereof.

**2.** Compounds of formula I as claimed in claim 1, wherein aryl denotes a phenyl or naphthyl residue which can optionally be substituted once or several times by lower alkyl, halogen, hydroxy, alkoxy, amino, dialkylamino, alkylmercapto, alkylsulfinyl, alkanesulfonyl, carboxamido, which can be substituted once or twice by lower alkyl, or by carboxyl, alkoxycarbonyl or cyano.

**3.** Compounds of formula I as claimed in claim 1, wherein the heterocyclic ring represents a 5 - 7-membered, saturated or unsaturated ring which can be substituted once or several times by halogen, $C_1$-$C_6$ alkyl or $C_1$-$C_6$ alkoxy, selected from the group comprising pyrrolidine, piperidine, azepine, tetrahydrofuran, tetrahydropyran, morpholine, dioxane, furan, thiophene, pyridine, pyrazole, imidazole, thiazole, oxazole, pyrimidine or pyrazine.

**4.** Compound as claimed in claim 3, wherein the heterocyclic ring is pyrrolidine, piperidine, morpholine, furan, thiophene, pyridine or imidazole.

**5.** Process for the production of compounds of formula I as claimed in claim 1 to 4, wherein a compound of formula II

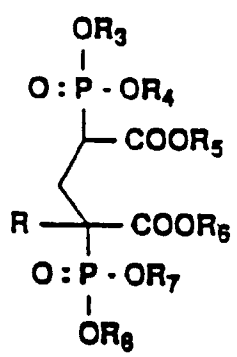

(II),

in which R has the aforementioned meaning and $R_3$, $R_4$, $R_5$, $R_6$, $R_7$ and $R_8$ each represent, independently of one another, $C_1$-$C_6$ alkyl, cycloalkyl, aryl or arylmethyl, is saponified in a well-known manner in such a way that either all ester bonds are hydrolyzed or partial saponification is achieved by suitable reagents

and subsequently, if desired, the optically active components are isolated from the diastereomers obtained and the obtained compounds are optionally converted into pharmacologically acceptable salts.

**6.** Compounds of formula II

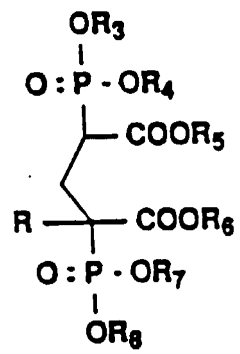

(II)

in which R has the aforementioned meaning and $R_3$, $R_4$, $R_5$, $R_6$, $R_7$ and $R_8$ each denote, independently of one another, $C_1$-$C_6$ alkyl, cycloalkyl, aryl or arylmethyl as well as their optically active compounds.

**7.** Process for the production of compounds of formula II as claimed in claim 6, wherein either

a) a compound of formula III

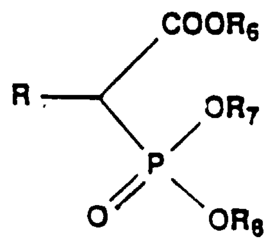

(III)

in which R, $R_6$, $R_7$ and $R_8$ have the stated meaning is reacted in a well-known manner with a compound of formula IV

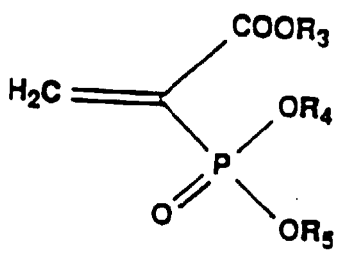

(IV),

IV

in which $R_3$, $R_4$ and $R_5$ have the stated meaning or

b) a compound of formula V

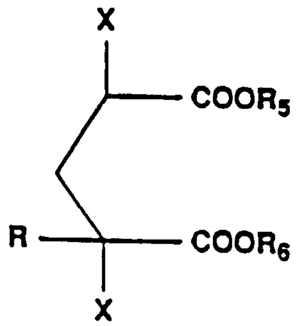

(V) ,

in which R, $R_5$ and $R_6$ have the stated meaning and X represents a leaving group is reacted in a well-known manner with a trialkylphosphite

and subsequently the diastereomers obtained are converted into the optically active components.

**8.** Pharmaceutical agent containing a compound of formula I as claimed in claims 1 to 4 in addition to common vehicles and auxiliary substances.

**9.** Use of compounds of formula I as claimed in claim 1, 2, 3 or 4 for producing pharmaceutical agents for the treatment of diseases of calcium metabolism.

## Revendications

**1.** Composés de formule I

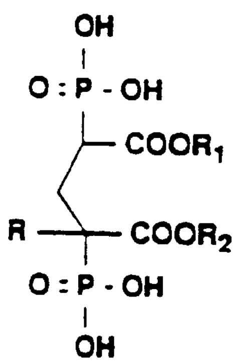

(I),

dans laquelle

R représente un groupe alkyle en $C_1$-$C_6$, pouvant éventuellement être substitué par un groupe hydroxy, alcoxy en $C_1$-$C_6$, amino, di(alkyle en $C_1$-$C_6$)amino, (alkyle en $C_1$-$C_6$)mercapto, (alkyle en $C_1$-$C_6$)sulfinyle, (alcane en $C_1$-$C_6$)sulfonyle, cycloalkyle en $C_3$-$C_7$, aryle ou un noyau hétérocyclique, ou R représente un groupe alcényle en $C_3$-$C_6$, cycloalkyle en $C_3$-$C_7$, cycloalcényle en $C_5$-$C_7$, aryle ou un noyau hétérocyclique et

$R_1$ et $R_2$ peuvent être chacun indépendamment un atome d'hydrogène, un groupe alkyle en $C_1$-$C_6$, cycloalkyle en $C_3$-$C_7$, aryle ou arylméthyle,

les composés optiquement actifs correspondants ainsi que leurs sels pharmacologiquement acceptables.

**2.** Composés de formule I selon la revendication 1, dans lesquels le terme aryle signifie un résidu phényle ou naphtyle, pouvant être éventuellement substitué une ou plusieurs fois par un groupe alkyle inférieur, un atome d'halogène, un groupe hydroxy, alcoxy, amino, dialkylamino, alkylmercapto, alkylsulfinyle, alcanesulfonyle, carboxamido pouvant éventuellement porter un ou deux substituants alkyle inférieur, ou par un groupe carboxyle, alcoxycarbonyle ou cyano.

3. Composés de formule I selon la revendication 1, dans lesquels le noyau hétérocyclique est un noyau saturé ou insaturé à 5-7 maillons, pouvant être substitué une ou plusieurs fois par un atome d'halogène, un groupe alkyle en $C_1$-$C_6$ ou alcoxy en $C_1$-$C_6$, choisi dans le groupe constitué par la pyrrolidine, la pipéridine, l'azépine, le tétrahydrofurane, le tétrahydropyrane, la morpholine, le dioxane, le furane, le thiophène, la pyridine, le pyrazole, l'imidazole, le thiazole, l'oxazole, la pyrimidine ou la pyrazine.

4. Composé selon la revendication 3, dans lequel le noyau hétérocyclique est la pyrrolidine, la pipéridine, la morpholine, le furane, le thiophène, la pyridine ou l'imidazole.

5. Procédé de préparation des composés de formule I selon les revendications 1 à 4, caractérisé en ce que l'on saponifie, de manière connue en soi, un composé de formule II

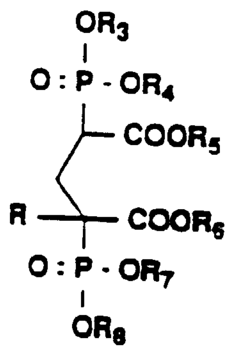

(II),

dans laquelle R est tel que défini ci-dessus et $R_3$, $R_4$, $R_5$, $R_6$, $R_7$ et $R_8$ représentent chacun indépendamment un groupe alkyle en $C_1$-$C_6$, cycloalkyle, aryle ou arylméthyle, de façon à ce que toutes les liaisons esters soient hydrolysées ou de façon à obtenir une saponification partielle à l'aide de réactifs appropriés,
puis, si on le souhaite, on isole les composants optiquement actifs à partir des diastéréoisomères obtenus et, éventuellement, on transforme les composés obtenus en sels pharmacologiquement acceptables.

6. Composés de formule II

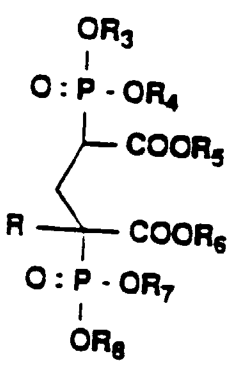

(II),

dans laquelle R est tel que défini ci-dessus et $R_3$, $R_4$, $R_5$, $R_6$, $R_7$ et $R_8$ représentent chacun indépendamment un groupe alkyle en $C_1$-$C_6$, cycloalkyle, aryle ou arylméthyle, ainsi que les composés optiquement actifs correspondants.

7. Procédé de préparation des composés de formule II selon la revendication 6, caractérisé en ce que l'on fait réagir de manière connue en soi, soit

a) un composé de formule III

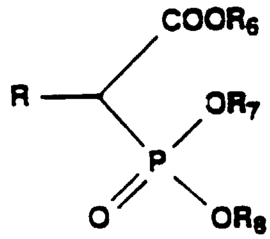

(III),

dans laquelle R, $R_6$, $R_7$ et $R_8$ ont les significations mentionnées, avec un composé de formule IV

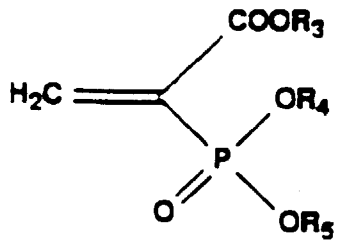

(IV),

dans laquelle $R_3$, $R_4$ et $R_5$ ont les significations mentionnées, soit

b) un composé de formule V

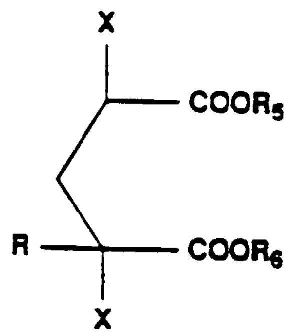

(V),

dans laquelle R, $R_5$ et $R_6$ ont les significations mentionnées et X représente un groupe partant, avec un trialkylphosphite,

puis, on transforme les diastéréoisomères obtenus en les composants optiquement actifs.

**8.** Médicament, contenant un composé de formule I selon les revendications 1 à 4 en plus de véhicules et adjuvants usuels.

**9.** Utilisation de composés de formule I selon la revendication 1, 2, 3 ou 4, pour la préparation de médicaments destinés au traitement des troubles du métabolisme calcique.